# FASCICULE DE BREVET EUROPEEN

(11) **EP 1 973 583 B1**
(45) Date de publication et mention de la délivrance du brevet: **14.09.2016**
(21) Numéro de dépôt: 06847105.1
(22) Date de dépôt: 21.12.2006
(51) Int. Cl.: A61L 27/54, A61L 27/28, B01J 20/32, A01N 57/00, A01N 59/00, A01N 59/16, A01N 33/12, A01P 1/00

(54) **PREPARATION D'UN SUBSTRAT INORGANIQUE PRESENTANT DES PROPRIETES ANTI-MICROBIENNES**
HERSTELLUNG EINES ANORGANISCHEN SUBSTRATES AUFWEISEND ANTIMIKROBIELLE EIGENSCHAFTEN
PREPARATION OF AN INORGANIC SUBSTRATE HAVING ANTIMICROBIAL PROPERTIES

(30) Priorité: 13.01.2006 FR 0600291
(43) Date de publication de la demande: 01.10.2008
(73) Titulaire: Centre National de la Recherche Scientifique, 75016 Paris (FR); Université de Montpellier II Sciences et Techniques du Languedoc, 34095 Montpellier (FR)
(72) Inventeur: MUTIN, Hubert, F-34830 Clapiers (FR); GUERRERO, Gilles, F-34500 Beziers (FR); AMALRIC, Julien, F-66740 Laroques Des Alberes (FR)
(74) Mandataire: Goulard, Sophie
(86) Numéro de dépôt international: PCT/FR2006/002828
(87) Numéro de publication internationale: WO 2007/080291

(56) Documents cités:
- EP-A- 1 157 994
- EP-A- 1 180 396
- EP-A- 1 488 815
- EP-A- 1 493 452
- WO-A-03/039602
- WO-A-03/041754
- WO-A-03/099346
- DE-A1- 10 211 562
- US-A- 4 962 073

## Description

La présente invention concerne un procédé de préparation d'un substrat inorganique présentant des propriétés anti-microbiennes ainsi qu'un substrat obtenu par ce procédé et l'utilisation d'un tel substrat.

Les micro-organismes tels que les bactéries sont susceptibles de se développer en s'associant à la surface de substrats inorganiques, en formant un biofilm. Ce mode de développement est directement impliqué dans un grand nombre d'infections bactériennes, qui peuvent survenir par exemple dans le domaine de l'industrie alimentaire, par l'intermédiaire des tuyauteries, ou dans le domaine médical, lors de la pose de prothèses ou d'implants.

Pour empêcher la formation de biofilms, il est connu de modifier la surface des substrats inorganiques en greffant des molécules à propriétés anti-microbiennes, ou des molécules libérant des groupes à propriétés anti-microbiennes. Les agents de couplage organosiliciés sont utilisés à cet effet depuis plusieurs années.

Toutefois, les agents organosiliciés ne peuvent pas être utilisés pour tous les types de substrats. Par exemple, ils ne conviennent pas aux substrats de type phosphate ou carbonate de calcium. En outre, le procédé de formation de tels revêtements est compliqué du fait que ces agents organosiliciés sont sensibles à l'humidité. De plus, les revêtements obtenus avec ces agents de couplage sont sensibles à l'humidité, en particulier en milieu basique.

Pour pallier ces inconvénients, les inventeurs ont trouvé qu'il était possible d'utiliser des agents de couplage organophosphorés pour lier à différents substrats des groupements possédant des propriétés anti-microbiennes ou susceptibles de libérer un élément possédant des propriétés anti-microbiennes.

Cette solution présente de nombreux avantages par rapport aux techniques de l'art antérieur.

Tout d'abord, il est possible de traiter par les mêmes agents de couplage une très grande variété de substrats (métaux et alliages métalliques, carbonates et phosphates métalliques). Cela permet de répondre notamment aux besoins dans le domaine médical, où jusqu'à présent aucune solution générale satisfaisante n'avait été trouvée pour diminuer les risques d'infection consécutifs à la pose de prothèses ou d'implants, en particulier à cause de la très grande diversité des matériaux utilisés. En outre, il est ainsi possible de traiter un objet complexe constitué de matériaux différents, ou de traiter des objets d'une même famille constitués chacun par un seul matériau qui peut varier d'un objet à l'autre.

Par ailleurs, le procédé de greffage peut être mis en oeuvre dans des conditions plus aisées que celles de l'art antérieur, par exemple à l'air ambiant, en conditions non anhydres voire même dans l'eau, ce qui permet d'éviter l'usage de solvants organiques.

Il est également possible de préparer des revêtements de surface très stables en présence d'eau, et même à pH basique, permettant une stérilisation par autoclavage humide ou un lavage par un désinfectant basique comme l'eau de Javel.

La recherche et la mise au point d'une solution pour l'élaboration de revêtements d'objets destinés à être utilisés dans le domaine médical peuvent être transposées à l'élaboration d'objets industriels, tels que des robinets ou des vannes constitués de plusieurs matériaux, ou des objets constitués par des matériaux pouvant varier, tels que des tuyauteries en acier, en acier zingué ou en cuivre, ces objets étant par exemple destinés à être utilisés dans des tours de refroidissement ou des circuits de distribution d'eau chaude. La possibilité de travailler dans l'eau permet d'envisager un traitement par simple circulation, dans une installation existante, d'une solution diluée de l'agent de couplage dans l'eau. EP1180396 décrit le revêtement de surface d'oxydes métalliques par des groupes fonctionnalisés utilisant des groupes phosphorés pour effectuer le couplage.

Ainsi, selon un premier aspect, l'invention concerne un procédé de modification d'un substrat inorganique visant à lui conférer des propriétés anti-microbiennes. Ledit procédé consiste à greffer en une ou plusieurs étapes sur une surface dudit substrat des groupements ayant des propriétés anti-microbiennes intrinsèques ou des groupements capables de libérer des espèces ayant des propriétés anti-microbiennes, et il est caractérisé en ce que le greffage est effectué par l'intermédiaire d'un agent de couplage organophosphoré qui est choisi parmi les acides phosphoniques et les phosphonates de formule (I) RPO(OX)₂, les bis-phosphonates de formule (II) RR'[PO(OX)₂]₂, les acides phosphiniques et les phosphinates de formule (III) RR'PO(OX), les monoalkyle phosphates de formule (IV) ROPO(OX)₂, et les dialkyle phosphates de formule (V) (RO)(R'O)PO(OX), dans lesquelles :
- X représente un atome d'hydrogène ou un groupe choisi parmi les ions métalliques, les ions ammonium, les groupements alkyle ou aryle comportant de 1 à 6 atomes de carbone et les groupements trialkylsilyles comportant de 1 à 6 atomes de carbone ;
- R est un groupement organique choisi parmi :
   - les groupements qui ont des propriétés anti-microbiennes intrinsèques ;
   - les groupements qui sont capables de libérer des espèces ayant des propriétés anti-microbiennes choisis parmi les groupements libérant du monoxyde d'azote et les groupements libérant des acides carboxyliques ;
   - les groupements R" qui, après modification, ont des propriétés anti-microbiennes intrinsèques ou sont capables de libérer des espèces ayant des propriétés anti-microbiennes, lesdits groupements R" étant choisis parmi les groupements -(CH₂)ₙ-SH, -(CH₂)ₙ-CN, -(CH₂)ₙ-NH₂, - (CH₂)ₙ-[NH- (CH₂)_{n'}]_{n"}-NH₂, - (CH₂)ₙ-NMe₂ , - (CH₂)ₙ-Hal, Hal représentant un atome d'halogène tel que Br, Cl, I, et dans lesquels n est compris entre 1 et 20, n' est compris entre 1 et 5 et n" est compris entre 1 et 10;
- R' est un atome d'hydrogène, un groupement hydroxyle ou un groupement organique répondant à la définition donnée pour les groupements R ci-dessus.

Parmi les groupements qui ont des propriétés anti-microbiennes intrinsèques, on peut citer les groupements comportant une ou plusieurs fonctions ammonium, pyridinium, imidazolium, phosphonium, sulfate, biguanide, carbanilide, amidine, pyrimidine, hydroxyquinoléine, acide carboxylique, des peptides ou des enzymes.

Selon une réalisation, l'agent de couplage est le 3-(bromure de N-méthyl imidazolium) propylphosphonate de diéthyle (BMImPPE). Dans ce cas, le composé répond à la formule (I) dans laquelle R = BrMeIm-(CH₂₎₃-, et X est un groupement éthyle.

Selon une autre réalisation, l'agent de couplage est l'acide 3-(bromure de N-méthyl imidazolium) propylphosphonique (BMImPPA). Dans ce cas, le composé répond à la formule (I) dans laquelle R = BrMeIm-(CH₂)₃-, et X est un atome d'hydrogène.

Parmi les groupements qui sont capables de libérer du monoxyde d'azote, on peut citer les groupements diazénium diolates ou oxynitroxy, et parmi les groupements capables de libérer des acides carboxyliques, on peut citer les groupements esters ou amides.

Lorsque R est du type R", le procédé comprend une ou plusieurs étapes supplémentaires.

Par exemple, si R" est -(CH₂)ₙ-SH, -(CH₂)ₙ-CN, -(CH₂)ₙ-NH₂ ou tout autre groupement complexant, la modification peut être effectuée par immersion dans une solution de nanoparticules d'argent ou dans une solution de AgNO₃, de sorte à permettre la libération d'ions Ag⁺ en milieu physiologique.

Si R" est - (CH₂)ₙ-NH₂, ou - (CH₂)ₙ-NH-(CH₂)₂-NH₂, la modification peut être effectuée par mise sous atmosphère de monoxyde d'azote gazeux, de sorte à permettre la libération de monoxyde d'azote en milieu physiologique.

Si R" est -(CH₂)ₙ-NMe₂, la modification peut être effectuée par chauffage dans une solution de bromure d'alkyle, de sorte à permettre la formation de fonctions ammonium quaternaire qui ont des propriétés anti-microbiennes intrinsèques.

Si R" est -(CH₂)ₙ-Hal avec Hal = Cl, Br, I, la modification peut être effectuée par chauffage dans une solution de triéthyle amine, de sorte à permettre la formation de fonctions ammonium quaternaire.

Selon une réalisation, l'agent de couplage est l'acide 12-mercaptododécylphosphonique (MDPA). Ce composé répond à la formule (I) dans laquelle R = R" = -(CH₂)₁₂-SH, et X est un atome d'hydrogène.

Selon une autre réalisation, l'agent de couplage est l'acide 2-aminoéthylphosphonique (AEPA). Ce composé répond à la formule (I) dans laquelle R = R" = -(CH₂)₂-NH₂, et X est un atome d'hydrogène.

Selon une autre réalisation, l'agent de couplage est le diéthyle 3-aminopropylphosphonate (APPE). Ce composé répond à la formule (I) dans laquelle R = R" = -(CH₂)₃-NH₂, et X est un groupement éthyle.

Selon une autre réalisation, l'agent de couplage est l'acide 12-bromododécylphosphonique (BDPA). Ce composé répond à la formule (I) dans laquelle R = R" = -(CH₂)₁₂-Br, et X est un atome d'hydrogène.

Selon une autre réalisation, l'agent de couplage est le 12-N-(aminoéthyl)-aminododécylphosphonate de diéthyle (AEADPE). Ce composé répond à la formule (I) dans laquelle R = R" = -(CH₂)₁₂-NH-(CH₂)₂-NH₂, et X est un groupement éthyle.

Selon une autre réalisation, l'agent de couplage est le 3-(N,N-diméthylamino) dodécylphosphonate de diéthyle (DMADPE). Ce composé répond à la formule (I) dans laquelle R = R" = -(CH₂)₁₂-N(Me)₂, et X est un groupement éthyle.

Le substrat à traiter peut être choisi parmi les métaux (par exemple un acier inoxydable, un acier galvanisé, du titane, du titane recouvert d'hydroxyapatite, un alliage à base de titane ou de chrome, de l'aluminium, du cuivre) un carbonate métallique (par exemple un carbonate de calcium), un phosphate métallique (par exemple de l'hydroxyapatite). Il peut être sous forme massive (plaque, feuille, pièce usinée ou moulée, métallique ou céramique) ou sous forme de poudre, par exemple une poudre d'hydroxyapatite ou de carbonate de calcium. Lorsque le substrat est sous forme de poudre, il peut être avantageusement utilisé, une fois modifié par le procédé selon l'invention, comme charge dans des polymères organiques ou pour entrer dans la fabrication de revêtements.

L'agent de couplage est greffé sur la surface dudit substrat de préférence par immersion dudit substrat dans une solution contenant ledit agent de couplage.

Le solvant utilisé pour l'agent de couplage peut être l'eau, l'alcool, un mélange eau-alcool, ou un solvant organique, par exemple le toluène, le dichlorométhane, le chloroforme, le DMSO, le DMF, le THF.

La concentration de l'agent de couplage est généralement comprise entre 0,1 et 100 mM. Lorsque le substrat est sous forme de plaque (très faible surface spécifique), la concentration de l'agent de couplage est de préférence comprise entre 0,1 et 10 mM, par exemple 0,5 ou 1 mM. Lorsque le substrat est sous forme de poudre, la concentration de l'agent de couplage est de préférence comprise entre 5 et 80 mM, en fonction de la surface spécifique.

Le pH de la solution contenant l'agent de couplage est choisi en fonction du substrat utilisé, de sorte à éviter toute dissolution du substrat.

Selon un deuxième aspect, l'invention a pour objet un substrat inorganique modifié obtenu par le procédé selon l'invention. Ledit substrat a une surface à laquelle sont liés des groupes organophosphorés ayant au moins un substituant organique R¹, ledit substituant organique R¹ possédant des propriétés anti-microbiennes ou étant susceptible de libérer, en milieu physiologique, une espèce possédant des propriétés anti-microbiennes.

Les groupes organophosphorés sont liés à la surface du substrat par l'intermédiaire de liaisons M-O-P dans lesquelles M représente le métal du substrat. Ces liaisons M-O-P proviennent de la condensation des groupements P-OX et/ou de la coordination des groupements phosphoryle P=O.

Les groupes organophosphorés sont de type phosphonate de formule R¹PO₁₋ₓ(OX)_{2-y}(OM)_{x+y}, bis-phosphonate de formule R¹R² [PO₁₋ₓ (OX)_{2-y}(OM)_{x+y}]₂, phosphinate de formule R¹R²PO₁₋ₓ(OX)_{1-z}(OM)_{x+z} , ou phosphate de formule R¹OPO₁₋ₓ(OX)_{2-y}(OM)_{x+y}, ou diester (R¹O)(R²O)POR¹R²PO₁₋ₓ(OX)_{1-z}(OM)_{x+z}, dans lesquels :
- X représente un atome d'hydrogène ou un groupe choisi parmi les ions métalliques, les ions ammonium, les groupements alkyle ou aryle comportant de 1 à 6 atomes de carbone et les groupements trialkylsilyles comportant de 1 à 6 atomes de carbone ;
- R¹ est un groupement organique choisi parmi :
   - les groupements qui ont des propriétés anti-microbiennes intrinsèques ;
   - les groupements qui sont capables de libérer des espèces ayant des propriétés anti-microbiennes, lesdits groupements étant choisis parmi les groupements libérant des ions métalliques, les groupements libérant du monoxyde d'azote ou les groupements libérant des acides carboxyliques ;
- R² est un atome d'hydrogène, un groupement hydroxyle ou un groupement organique pouvant éventuellement faire partie des groupements R¹ définis ci-dessus ;
- M représente le métal du substrat ;
- x vaut 0 ou 1 ;
- y vaut 0, 1, ou 2 ;
- Z vaut 0 ou 1.

Le substrat peut être un métal, un carbonate métallique, un phosphate métallique, portant à sa surface des groupements possédant des propriétés anti-microbiennes ou des groupements susceptibles de libérer des éléments possédant des propriétés anti-microbiennes choisis parmi les groupements libérant des ions métalliques, les groupements libérant du monoxyde d'azote ou les groupements libérant des acides carboxyliques, ces groupements étant liés par l'intermédiaire d'un groupe organophosphoré tel que défini ci-dessus.

Parmi les groupements qui ont des propriétés anti-microbiennes intrinsèques, on peut citer les groupements comportant une ou plusieurs fonctions ammonium, pyridinium, imidazolium, phosphonium, sulfate, biguanide, carbanilide, amidine, pyrimidine, hydroxyquinoléine, acide carboxylique, des peptides, des enzymes.

Parmi les groupements libérant des ions métalliques (argent, or, cuivre, zinc), on peut citer des groupements portant une ou plusieurs fonctions amine, acide, thiol, cyano, ou disulfure, complexant des ions ou des particules métalliques. Parmi les groupements libérant du monoxyde d'azote, on peut citer les groupements diazénium diolates ou oxynitroxy, et parmi les groupements libérant des acides carboxyliques, on peut citer les groupements esters ou amides.

Dans le substrat selon l'invention, le groupement R¹ peut être un groupement - (CH₂)₁₂-SH ayant fixé des ions Ag⁺, un groupement -(CH₂)₁₂-SH ayant fixé des particules d'argent, un groupement -(CH₂)₁₂-Br ayant fixé une amine tertiaire pour former un ammonium quaternaire, un groupement -(CH₂)₂-NMe₂ ayant fixé du bromure d'alkyle pour former un ammonium quaternaire, un groupement -(CH₂)₂-NH₂ ayant fixé du monoxyde d'azote pour former un groupement diazéniumdiolate, un groupement -(CH₂)₃-NH₂ ayant fixé du monoxyde d'azote, un groupement - (CH₂)₁₂-NH-(CH₂)₂-NH₂ ayant fixé du monoxyde d'azote, un groupement - (CH₂)₃-C₃H₃N₂MeBr.

Le substrat modifié est, selon une réalisation, constitué d'une feuille de titane modifiée par MDPA et des ions Ag⁺.

Selon une autre réalisation, le substrat modifié est constitué d'une feuille de titane modifiée par MDPA et des particules d'argent.

Selon une autre réalisation, le substrat modifié est constitué d'une feuille d'acier inoxydable modifiée par MDPA et des ions Ag⁺.

Selon une autre réalisation, le substrat modifié est constitué d'une feuille de titane modifiée par AEPA et du monoxyde d'azote NO.

Selon une autre réalisation, le substrat modifié est constitué d'une feuille d'acier inoxydable modifiée par AEPA et du monoxyde d'azote NO.

Selon une autre réalisation, le substrat modifié est constitué d'une feuille de titane modifiée par APPE et du monoxyde d'azote NO.

Selon une autre réalisation, le substrat modifié est constitué d'une feuille d'acier inoxydable modifiée par APPE et du monoxyde d'azote NO.

Selon une autre réalisation, le substrat modifié est constitué d'une feuille de titane modifiée par BMImPPE.

Selon une autre réalisation, le substrat modifié est constitué d'une feuille d'acier inoxydable modifiée par BMImPPE.

Selon une autre réalisation, le substrat modifié est constitué d'une feuille d'acier inoxydable modifiée par BMImPPA.

Selon une autre réalisation, le substrat modifié est constitué d'une feuille de titane modifiée par AEADPE et du monoxyde d'azote NO.

Selon une autre réalisation, le substrat modifié est constitué d'une feuille d'acier inoxydable modifiée par BDPA et de la triéthylamine Et₃N.

Selon une autre réalisation, le substrat modifié est constitué d'une poudre d'hydroxyapatite modifiée par BMImPPE.

Selon une autre réalisation, le substrat modifié est constitué d'une poudre de carbonate de calcium modifiée par BMImPPE.

Selon une autre réalisation, le substrat modifié est constitué d'une poudre d'hydroxyapatite modifiée par BDPA puis de la triéthylamine.

Selon une autre réalisation, le substrat modifié est constitué d'une poudre de carbonate de calcium modifiée par BDPA puis de la triéthylamine.

L'invention concerne également l'utilisation d'un substrat selon l'invention dans des pièces telles que des implants métalliques ou céramiques, des instruments chirurgicaux, des échangeurs de chaleur, des éléments de tuyauterie, ou des pièces formées de matériaux composites polymère-charge, dans lesquels la charge est modifiée par le procédé selon l'invention.

La présente invention est illustrée ci-après par des exemples concrets de réalisation.

### Exemple 1

### Préparation d'une feuille de titane modifiée par MDPA et des nanoparticules d'argent

### Préparation du MDPA

Le MDPA a été synthétisé en trois étapes. Dans la première étape, on prépare le 12-bromo dodécylphosphonate de diéthyle par réaction du 1-12 dibromododécane (40 g, 120,6 mmoles) avec la triéthyle phosphite (26 ml /150 mmoles) à 150°C pendant 12 heures sous argon. Après refroidissement on ajoute 100 mL d'eau distillée. La phase organique est extraite, lavée avec 50 mL d'eau distillée, séchée sur sulfate de sodium, filtrée et concentrée sous pression réduite. Le produit obtenu, une huile de couleur jaune pâle, est purifié par chromatographie sur colonne de gel de silice (éluant hexane/AcOEt 8%) pour fournir 20 g (rendement 43%) de 12-bromo dodécylphosphonate de diéthyle.

Dans la deuxième étape on fait réagir sous atmosphère inerte le 12-bromododécylphosphonate de diéthyle (20,01 g; 51,9 mmoles) avec la thiourée (4 g; 51,9 mmoles) dans 200 ml d'eau à 100°C sous agitation pendant 12 heures. Après refroidissement, on ajoute goutte à goutte une solution de soude (2,07 g / 51,9 mmoles) dans 100 ml d'eau et le mélange réactionnel est chauffé au reflux pendant 3 heures. Après retour à température ambiante, une solution d'acide chlorhydrique (32%) est additionnée goutte à goutte jusqu'à pH = 1. La solution est ensuite laissée sous agitation pendant 24 heures. La phase organique est alors récupérée avec 100 ml de CH₂Cl₂, lavée à l'eau distillée, séchée sur sulfate de sodium, filtrée et évaporée sous pression réduite. L'huile de couleur jaune obtenue est purifiée par chromatographie sur colonne de gel de silice (éluant hexane/AcOEt 8%) pour fournir 10,8 g (n=38,16 mmoles) de 12-mercaptododécylphosphonate de diéthyle (rendement 52%).

Dans la troisième étape on fait réagir sous atmosphère inerte 2,4 g (6,2 mmoles) de mercaptododécylphosphonate de diéthyle dans 50 ml de CH₂Cl₂ sec avec 2,4 ml (18 mmoles) de bromotriméthylsilane sous agitation pendant 3 heures à température ambiante. La solution obtenue est ensuite concentrée sous pression réduite puis on ajoute 50 ml de CH₂Cl₂ et 1,7 mL d'eau distillée. Après agitation pendant 12 heures à température ambiante, évaporation du solvant et recristallisation dans un mélange CH₂Cl_{2/}Hexane, on obtient 1,8 g de MDPA (rendement 90%).

### Caractérisation du MDPA

**RMN ¹H (250 MHz, D₂O, ppm) :** 1,30-1,67(m, 22H, (C**H**₂)₁₁CH₂SH)), 2,55 (q, 2H, CH₂SH)
**RMN** ¹³**C (100 MHz, CDCl₃,ppm) :** 21,7 (d, 8Hz, **C**H₂CH₂CH₂P), 23,3 (d, 5Hz, **C**H₂CH₂P), 24,7 (s, **C**H₂SH), 28,1 (s, **C**H₂CH₂CH₂SH), 28,1 (d, 5Hz, **C**H₂-(CH₂)₃-P), 28,5 (S,**C**H₂-(CH₂)₃SH), 29 (**C**H₂-(CH₂)₄SH), 29, 1 (s, **C**H₂-(CH₂)₄P), 29,2 (s, **C**H₂-(CH₂)₅-P), 29,1 (s, **C**H₂-(CH₂)₆-P), 33,2 (d, 14Hz, **C**H₂-P) 33,9 (s, 2H, **C**H₂CH₂SH).
**RMN** ³¹**P (101 MHz, CDCl₃, ppm) :** 31,2.

### Préparation d'une solution de nanoparticules d'argent

Une solution de nanoparticules d'argent est préparée en dissolvant 0,5 g de nitrate d'argent dans 40 g d'eau déionisée contenant 0,1% en poids de Tween 80. 10 g d'une solution d'hydrazine monohydrate à 0,05 % dans l'eau déionisée sont ajoutés au goutte à goutte. On complète ensuite à 100 g avec de l'eau déionisée. Le mélange est agité à température ambiante pendant 6 heures, puis la suspension est centrifugée afin d'éliminer les particules les plus grosses.

### Préparation de la feuille de titane modifiée

Une feuille de titane (fournisseur Aldrich), de pureté 99,7%, d'épaisseur 0,127 mm, et dont les dimensions sont 1,8 cm x 1,8 cm, est nettoyée aux ultrasons dans du pentane pendant 4 minutes puis traitée sous UV-ozone pendant 30 minutes.

La feuille est ensuite immergée dans 5 ml d'une solution de MDPA dans de l'éthanol absolu, à une concentration de 1 mM, pendant 24 heures à 25°C.

Après réaction, la feuille est lavée abondamment avec le solvant de réaction, puis rincée successivement avec de l'éthanol, de l'eau et du chloroforme.

La feuille de titane modifiée par MDPA est immergée pendant 15 heures dans 5 ml de la solution de nanoparticules d'argent, puis elle est rincée successivement à l'éthanol, à l'eau et au chloroforme. Une analyse par spectroscopie de photoélectrons confirme la présence d'argent à la surface de l'échantillon.

### Exemple 2

### Préparation d'une feuille de titane modifiée par MDPA et des ions Ag⁺

### Préparation du MDPA

Le MDPA est préparé selon le mode opératoire décrit dans l'exemple 1.

### Préparation de la feuille de titane modifiée

Une feuille de titane (fournisseur Aldrich, de pureté 99,7%, d'épaisseur 0,127 mm, et de dimensions 1 cm x 1 cm), est modifiée par MDPA dans les mêmes conditions opératoires que pour l'exemple 1.

Cette feuille est ensuite immergée pendant 2 heures dans 5 ml d'une solution de AgNO₃ de concentration 1 mM dans l'eau déionisée, puis elle est rincée successivement à l'éthanol, à l'eau et au chloroforme.

### Exemple 3

### Préparation d'une feuille de titane modifiée par AEPA et du monoxyde d'azote

### Préparation de la feuille de titane modifiée

Une feuille de titane est modifiée par AEPA (commercialisé par la société Aldrich) dans les mêmes conditions opératoires que pour l'exemple 2, en remplaçant la solution de MDPA par une solution aqueuse de AEPA de concentration 1mM.

Cette feuille est placée pendant 3 jours à 25°C dans un réacteur sous 5 bars de NO. Le réacteur est ensuite purgé avec de l'argon sec.

### Exemple 4

### Préparation d'une feuille de titane modifiée par APPE et du monoxyde d'azote

### Préparation du composé APPE

Le composé APPE est synthétisé en deux étapes.

Dans la première étape, la réaction du 3-bromopropylphtalimide avec la triéthyle phosphite conduit au phtalimidopropylphosphonate de diéthyle. A cet effet, dans un ballon de 250 ml contenant 10 g de triéthylphosphite, on ajoute 24,22 g de bromopropylphtalimide. Le mélange est porté à 140°C pendant 12 heures sous atmosphère d'argon. Le mélange est alors dégazé sous pression réduite.

Le produit est purifié par chromatographie sur colonne de silice avec le dichlorométhane comme éluant puis un gradient de méthanol (5% puis 10%). On récupère 17,33 g de phtalimidopropylphosphonate de diéthyle avec un rendement de 59%.

### Caractérisation du phtalimidopropylphosphonate de diéthyle

**Rf :** 0,20 (CH₂Cl₂)
**RMN** ¹**H** (250 MHz, CDCl₃, ppm) : 1,33 (m, 6H, CH₃CH₂O), 1,74-2,03 (m, 4H, P-CH₂-CH₂CH₂-N), 3,78 (t, 2H, P-CH₂-CH₂CH₂-NH₂), 4,08 (m, 4H, CH₃CH₂O), 7,7-7,9 (m, 4H, cycle aromatique)
**RMN** ¹³**C** (100 MHz, CDCl₃, ppm) : 16,11-16,5 (m, P-OCH₂**C**H₃, N-CH₂-**C**H₂-CH₂-P), 22,68 (t, large, N-CH₂-CH₂-**C**H₂-P), 38,34 (d, N-**C**H₂-CH₂-CH₂-P), 61,45 (q, P-O**C**H₂CH₃), 123,29 (t, CHAr-**C**HAr=CAr), 132,05 (d, CHAr-CHAr=CAr), 134,05 (s, **C**Ar-C(O)-N), 168,26 (s, CAr-**C**(O)-N).
**RMN** ³¹**P** (101 MHz, CDCl₃, ppm) : 31,24
**Spectrométrie de masse(FAB+/GT) m/z** : 326 (M+1)

Dans la deuxième étape, le phtalimidopropylphosphonate de diéthyle est mis à réagir avec l'hydrazine monohydrate pour donner le composé APPE.

A cet effet, on ajoute à 15 g (46,1 mmol) de phtalimidopropylphosphonate de diéthyle, 23,08 g d'hydrazine monohydrate (46,1 mmol) au goutte-à-goutte dans environ 500 mL d'éthanol absolu. Le milieu réactionnel est agité pendant 12 heures. Les sels formés sont filtrés et lavés dans l'éthanol. Après évaporation de l'éthanol, on obtient de nouveau des sels. Ceux-ci sont lavés avec CH₂Cl₂ et filtrés sur membrane Millipore (0,45 µm). On récupère le filtrat et on évapore CH₂Cl₂. On obtient une huile jaune. Le produit brut est purifié par chromatographie sur colonne de gel de silice avec un mélange CHCl_{3/}MeOH à 90/10 comme éluant. On obtient 4,6 g de 3-aminopropylphosphonate de diéthyle (APPE) avec un rendement de 51,8%.

### Caractérisation du composé APPE

**RMN** ¹**H** (250 MHz, CDCl₃, ppm) : 1,33 (m, 6H, CH₃CH₂O), 1,64-1,87 (m, 4H, P-CH₂-CH₂CH₂-N), 2,77 (t, 2H, P-CH₂-CH₂CH₂-NH₂), 4,1 (m, 4H, CH₃CH₂O)
**RMN** ³¹**P** (101 MHz, CDCl₃, ppm) : 33,344

### Préparation de la feuille de titane modifiée

Une feuille de titane est modifiée par APPE dans les mêmes conditions opératoires que pour l'exemple 1, en remplaçant la solution de MDPA par une solution de APPE dans le chloroforme de concentration 1 mM, et en chauffant pendant 5 jours à 65°C.

Cette feuille est ensuite placée pendant 3 jours à 25°C dans un réacteur sous 5 bars de NO. Le réacteur est ensuite purgé avec de l'argon sec.

### Exemple 5

### Préparation d'une feuille de titane modifiée par BMImPPE

### Préparation de Br⁻ Me-C₃H₃N₂⁺-(CH₂)₃PO₃Et₂ (BMImPPE)

Ce composé est obtenu en deux étapes. Dans la première étape, on prépare du 3-bromopropylphosphonate de diéthyle, par réaction sous atmosphère inerte du 1-3 dibromopropane (90,9 g; 0,45 mol) avec la triéthyle phosphite (49,8 g; 0,3 mol) à 140°C pendant 12 heures, puis distillation sous pression réduite (rendement 52%).

Dans la deuxième étape on obtient BMImPPE par réaction du 3-bromopropylphosphonate de diéthyle avec le 1-méthylimidazole, selon le mode opératoire suivant. Dans un tricol sous argon contenant 50 mL de THF sec, sont ajoutés 3,18 g (38,6 mmol) de 1-méthylimidazole et 10,01 g (38,6 mmol) de 3-bromopropylphosphonate de diéthyle. Le milieu réactionnel est porté à 70°C pendant 12 heures pour conduire à un mélange biphasique. Après décantation, le liquide huileux résultant est lavé avec 2 x 50 mL de THF. L'huile orange obtenue est lavée une nouvelle fois avec 3 x 30 mL d'éther anhydre pour donner le produit attendu sous la forme d'une huile marron avec un rendement de 60%.

### Caractérisation du BMImPPE

**RMN** ¹**H** (δ, ppm, 200 MHz, D₂O) : 1,22 (t, 6H, O-CH₂-CH₃), 2,17-1,77 (m, 4H, CH₂-CH₂-P), 3,80 (s, 3H, CH₃-N), 4,04 (m, 4H, O-CH₂-CH₃), 4,23 (t, 2H, CH₂-N), 7,36 (d, 1H, N-CH), 7,41(d, 1H, N-CH), 8,67 (s, 1H, N-CH-N).
**RMN** ³¹**P** (δ, ppm, 81 MHz, D₂O) : 31,01.
**RMN** ¹³**C** (δ ppm, 100 MHz, D₂O) : 17,4 (O-CH₂-**C**H₃), 21,7 ppm (**C**H₂-CH₂-P), 23,1-24,3 (d, **C**H₂-P), 37,1 (**C**H₃-N), 50,2 (d, **C**H₂-N), 62,9 (d, **C**H₂-O), 123,4-124,8 (d, N-**C**H-**C**H-N), 137,7 (N-**C**H-N).

### Préparation d'une feuille de titane modifiée par Br⁻ MeC₃H₃N₂⁺- (CH₂) ₃-PO₃Et₂ (BMImPPE)

Une feuille de titane est modifiée par BMImPPE dans les mêmes conditions opératoires que pour l'exemple 4, en remplaçant la solution de APPE par une solution de BMImPPE de concentration 1mM dans le chloroforme.

### Exemple 6

### Préparation d'une feuille de titane modifiée par AEADPE et du monoxyde d'azote

### Préparation du composé AEADPE

Le composé AEADPE est synthétisé en deux étapes.

Dans la première étape, le 12-bromo dodécylphosphonate de diéthyle est préparé selon le mode opératoire décrit dans l'exemple 1.

Dans la deuxième étape, le 12-bromo dodécylphosphonate de diéthyle (3,85 g ; 10 mmoles) est ajouté goutte à goutte sous agitation à l'éthylène diamine (4,01 g ; 100 mmoles) à température ambiante. Après deux heures d'agitation à température ambiante, 100 mL d'acétate d'éthyle sont ajoutés et un milieu biphasique est obtenu. La phase inférieure est éliminée et la phase supérieure concentrée sous pression réduite. 60 mL d'acétate d'éthyle supplémentaires sont alors ajoutés et la solution placée une nuit au congélateur. Un milieu biphasique est obtenu. La phase inférieure est éliminée et la phase supérieure concentrée sous pression réduite. L'huile de couleur jaune obtenue est purifiée par chromatographie sur colonne de gel de silice avec l'acétate d'éthyle comme éluant puis avec un gradient d'éthanol pour fournir 3,6 g de 12-N-(aminoéthyl)-aminododécylphosphonate de diéthyle avec un rendement de 99%.

### Caractérisation du 12-N-(aminoéthyl)-aminododécylphosphonate de diéthyle

**Rf :** 0,05 (EtOH Abs.)
**RMN** ¹**H** (250 MHz, CDCl₃, ppm) : 1,09-1,97 (m, 31H, CH₃CH₂O, NH, NH₂, P(CH₂)₁₁), 2,54-2,68 (m, 4H, CH₂-NH₂, CH₂-NH), 2,79 (t, 2H, CH₂-NH), 4,07 (m, 4H, CH₂O).
**RMN** ¹³**C** (100 MHz, CDCl₃, ppm) : 16,9 (d, O**C**H₂), 22,8 (d, **C**H₂-CH₂-P), 24,7-27,4 (d, CH₂-P), 29,4-30,6 (m, P(CH₂)₃-(**C**H₂)₈)), 31,0 (d, **C**H₂-(CH₂)₂-P), 42, 2 (s, **C**H₂-NH₂), 50, 3 (s, **C**H₂-NH), 53 (s, HN-**C**H₂-CH₂-NH₂), 61, 7 (d, **C**H₂O).
**RMN** ³¹**P** (101 MHz, CDCl₃, ppm) : 33,9

### Préparation de la feuille de titane modifiée

Une feuille de titane est modifiée par AEADPE dans les mêmes conditions opératoires que pour l'exemple 4, en remplaçant la solution de APPE par une solution de AEADPE dans le chloroforme de concentration 1 mM.

Cette feuille est ensuite placée pendant 3 jours à 25°C dans un réacteur sous 5 bars de NO. Le réacteur est ensuite purgé avec de l'argon sec.

### Exemple 7

### Préparation d'une feuille d'acier inoxydable modifiée par MDPA et des ions Ag⁺

### Préparation du MDPA

Le MDPA est préparé selon le mode opératoire décrit dans l'exemple 1.

### Préparation de la feuille d'acier inoxydable modifiée

Une feuille d'acier inoxydable (fournisseur Goodfellow, référence AISI 316) d'épaisseur 0,15 mm et dont la composition est la suivante : Fe 69%, Cr 18%, Ni 10%, Mo 3%, est modifiée par MDPA dans les mêmes conditions que la feuille de titane de l'exemple 1.

Cette feuille est ensuite immergée pendant 2 heures dans 5 ml d'une solution de AgNO₃ de concentration 1 mM dans l'eau déionisée, puis elle est rincée successivement à l'eau, à l'éthanol et au chloroforme.

### Exemple 8

### Préparation d'une feuille d'acier inoxydable modifiée par AEPA et du monoxyde d'azote

Une feuille d'acier inoxydable identique à celle de l'exemple 7 est modifiée par AEPA et du monoxyde d'azote dans les mêmes conditions opératoires que la feuille de titane décrite dans l'exemple 3.

### Exemple 9

### Préparation d'une feuille d'acier inoxydable modifiée par APPE et du monoxyde d'azote

Une feuille d'acier inoxydable identique à celle de l'exemple 7 est modifiée par APPE et du monoxyde d'azote dans les mêmes conditions opératoires que la feuille de titane décrite dans l'exemple 4.

### Exemple 10

### Préparation d'une feuille d'acier inoxydable modifiée par BMImPPA

### Préparation de Br⁻ Me-C₃H₃N₂⁺-(CH₂)₃-PO₃H₂ (BMImPPA)

Ce composé est obtenu en deux étapes. Dans la première étape, le BMImPPE est préparé selon le mode opératoire décrit dans l'exemple 5.

Dans la deuxième étape on fait réagir sous atmosphère inerte 7,15 g (6,31 mmoles) de 3-(bromure de N-méthylimidazolium) propylphosphonate de diéthyle dans 40 ml de CH₂Cl₂ sec avec 2,5 ml (18,93 mmoles) de bromotriméthylsilane sous agitation pendant 12 heures à température ambiante. La solution obtenue est ensuite concentrée sous pression réduite puis on ajoute 40 ml de CH₂Cl₂ et 1,7 mL d'eau distillée. Après agitation pendant 12 heures à température ambiante et évaporation du solvant, l'huile obtenue est lavée à l'éther diéthylique. Après séparation des deux phases, l'huile est solubilisée dans de l'eau distillée et extraite à l'éther diéthylique. La phase aqueuse est alors concentrée sous pression réduite et on obtient 1,6 g de BMImPPA (rendement 89%).

### Caractérisation du BMImPPA

**RMN** ¹**H** (δ, ppm, 200 MHz, D₂O): 1,6 (m, 2H, CH₂-CH₂-CH₂-P), 2,0 (m, 2H, CH₂-P), 3,7 (s, 3H, CH₃-N), 4,15 (t, 2H, CH₂-N), 7,3 (d, 2H, N-CH-CH-N), 8,6 (s, 1H, N-CH-N).
**RMN** ³¹**P** (δ, ppm, 81 MHz, D₂O) : 30,35.
**RMN** ¹³**C** (δ, ppm, 100 MHz, D₂O) : 23,3 ppm (**C**H₂-CH₂-P), 23,5 (d, **C**H₂-P), 36,2 (s, **C**H₃-N), 49,7 (d, **C**H₂-N), 123,5 (d, N-**C**H-**C**H-N), 136,4 (N-**C**H-N).

### Préparation d'une feuille d'acier inoxydable modifiée par Br⁻ Me-C₃H₃N₂⁺-(CH₂) ₃-PO₃H₂ (BMImPPA)

Une feuille d'acier inoxydable identique à celle de l'exemple 7 est modifiée par BMImPPA dans les mêmes conditions opératoires que la feuille de titane décrite dans l'exemple 1.

### Exemple 11

### Préparation d'une feuille d'acier inoxydable modifiée par BMImPPE

Une feuille d'acier inoxydable identique à celle de l'exemple 7 est modifiée par BMImPPE dans les mêmes conditions opératoires que la feuille de titane décrite dans l'exemple 5. L'analyse de la surface par SIMS (Spectrométrie de masse d'ions secondaires) confirme le greffage (présence de carbone et d'azote).

### Exemple 12

### Préparation d'une feuille d'acier inoxydable modifiée par BDPA puis par la triéthylamine

### Préparation de l'acide 12-bromododécylphosphonique (BDPA)

Le composé BDPA a été synthétisé en deux étapes. Dans la première étape, on prépare le 12-bromo dodécylphosphonate de diéthyle comme précisé dans l'exemple 1. Dans la deuxième étape, le 12-bromo dodécylphosphonate de diéthyle (2,43 g; 6,32 mmoles) est mis en réaction avec Me₃SiBr (2,89 g; 18,93 mmoles) dans 50 ml de CH₂Cl₂ sec sous agitation à température ambiante pendant 12 heures.

Après concentration par évaporation sous pression réduite on ajoute 50 ml de CH₂Cl₂ sec puis 1,7 mL d'eau (0,095 mol). Le mélange obtenu est agité pendant 3 heures à température ambiante puis le solvant est évaporé et le BDPA est purifié par recristallisation dans CH₂Cl₂ sec (rendement 89 %).

### Caractérisation du BDPA

**RMN** ¹**H** (δ, ppm, 200 MHz, DMSO) : 1,2-1,6 (m, 20H, CH₂), 1,79 (q, 2H, CH₂-CH₂-Br), 3,53 (t, 3H, 2H, CH₂-Br).
**RMN** ³¹**P** (δ, ppm, 81 MHz, DMSO) : 27,87.

### Préparation d'une feuille d'acier inoxydable modifiée par BDPA

Une feuille d'acier inoxydable identique à celle de l'exemple 7 est modifiée par BDPA dans les mêmes conditions opératoires que la feuille d'acier inoxydable décrite dans l'exemple 10.

### Préparation d'une feuille d'acier inoxydable modifiée par BDPA puis par Et₃N

La feuille d'acier inoxydable modifiée par BDPA est chauffée à 78°C pendant 24 heures dans une solution de 2 mL (14,4 mmoles) de triéthylamine dans 11 mL d'éthanol absolu. Après retour à température ambiante, la feuille d'acier inoxydable est lavée à l'éthanol, puis rincée à l'eau, à l'éthanol et au chloroforme.

### Exemple 13 non représentatif de l'invention

### Préparation d'une poudre de TiO₂ modifiée par BMImPPE

### Préparation du composé BMImPPE

Le composé BMImPPE est préparé selon le mode opératoire décrit dans l'exemple 5.

### Préparation de la poudre de TiO₂ modifiée

Dans un ballon de 250 ml, on ajoute 150 ml de solution de BMImPPE dans le chloroforme, à une concentration de 1 mM, à 150 mg de TiO₂ (commercialisé par la société NORPRO, de surface spécifique 120 m²/g). Le mélange est porté à reflux du chloroforme pendant une semaine. Après retour à température ambiante, la solution est éliminée par filtration sur fritté Millipore. La poudre est ensuite lavée abondamment au chloroforme puis rincée successivement à l'eau, l'éthanol, au chloroforme et à l'acétone, puis séchée sous pression réduite à 120°C pendant 15 heures. La caractérisation de la poudre par RMN du solide confirme le greffage (³¹P, ppm: 26,5; ¹³C, ppm: 138,6; 125,0; 59,4; 52,3; 38,4; 26,6; 18,3).

### Exemple 14 non représentatif de l'invention

### Préparation d'une poudre de TiO₂ modifiée par BDPA puis par la triéthylamine

### Préparation de l'acide 12-bromododécylphosphonique (BDPA)

Le composé BDPA est préparé selon le mode opératoire décrit dans l'exemple 12.

### Préparation de la poudre de TiO₂ modifiée

Dans un ballon de 250 ml, on ajoute 200 ml d'une solution de BDPA dans l'éthanol absolu (concentration 1mM) à 200 mg de TiO₂ (commercialisé par NORPRO). Le mélange est agité trois jours à température ambiante. Après filtration et lavages à l'éthanol, cette poudre est ensuite chauffée au reflux d'un mélange de triéthylamine (2 mL) et d'éthanol (11 mL) pendant 24 heures. Après retour à température ambiante, la poudre est lavée à l'éthanol et à l'acétone, puis séchée sous pression réduite à 120°C pendant 15 heures. La caractérisation de la poudre par RMN ³¹P du solide confirme le greffage (pic large centré à 26,3 ppm).

### Exemple 15 non représentatif de l'invention

### Préparation d'une poudre de TiO₂ modifiée par DMADPE puis par le bromure d'éthyle

### Préparation du DMADPE

Le DMADPE est préparé par réaction de la diméthylamine avec le bromododécylphosphonate de diéthyle (préparé selon le mode opératoire décrit dans l'exemple 1).

2 g (5,19 mmol) de BDPE dans 25 ml d'acétonitrile sont ajoutés goutte à goutte à 0,24 g (5,19 mmol) de diméthylamine, 1,43 g (10,39 mmol) de K₂CO₃ et 75 ml d'acétonitrile chauffé à 90°C sous atmosphère inerte. Après 12 heures à 90°C, le mélange réactionnel est refroidi à température ambiante, les sels sont éliminés par filtration et le filtrat concentré pour donner une huile jaune. Cette huile est purifiée sur colonne de gel de silice (éluant AcOEt/MeOH, 85/15 à 70/30) pour donner le DMADPE.

### Caractérisation du DMADPE

RMN ³¹P (CDCl₃) : 33,19
RMN ¹H (CDCl₃) : 4,13 (m, O-CH₂, 4H) ; 2,62 (t, CH₂-N, 2H) ; 2,51 (s, N-CH₃, 6H) ; 1, 83-1, 25 (massif, (CH₂)₁₁ + OCH₂CH₃, 28H)

### Préparation de la poudre de TiO₂ modifiée

Dans un ballon de 250 ml, on ajoute 150 ml de solution de DMADPE dans le chloroforme, à une concentration de 1mM, à 150 mg de TiO₂. Le mélange est porté à reflux du chloroforme pendant une semaine. Après filtration et lavages au chloroforme puis à l'éthanol, cette poudre est ensuite chauffée au reflux d'un mélange de bromoéthane (5 mL) et d'éthanol (8 mL) pendant 24 heures. Après retour à température ambiante, le solide est ensuite lavé et séché comme dans l'exemple 13. La caractérisation de la poudre par RMN ³¹P du solide confirme le greffage (pic large centré à 26,1 ppm).

### Exemple 16

### Préparation d'une poudre d'hydroxyapatite modifiée par BMImPPE

### Préparation du BMImPPE

Le BMImPPE est synthétisé selon le mode opératoire décrit dans l'exemple 5.

### Préparation de la poudre d'hydroxyapatite modifiée

Dans un ballon de 250 ml, on ajoute 200 ml d'une solution de BMImPPE dans l'éthanol (concentration de 1 mM) à 360 mg d'hydroxyapatite (commercialisée par la société ACROS, surface spécifique 67 m²/g).

Le mélange est porté à reflux pendant 3 jours. Après retour à température ambiante, le solide est ensuite lavé et séché comme dans l'exemple 13. La caractérisation de la poudre par RMN ³¹P du solide confirme le greffage (pic large centré à 25,8 ppm en plus d'un signal à 2,6 ppm provenant des unités phosphates de l'hydroxyapatite).

### Exemple 17

### Préparation d'une poudre de carbonate de calcium modifiée par BMImPPE

### Préparation du BMImPPE

Le BMImPPE est synthétisé selon le mode opératoire décrit dans l'exemple 5.

### Préparation de la poudre de carbonate de calcium modifiée

Dans un ballon de 250 ml, on ajoute 100 ml d'une solution de BMImPPE dans l'éthanol (concentration de 1 mM) à 400 mg de CaCO₃ (surface spécifique 30 m²/g). Le mélange est agité trois jours à température ambiante. Le solide est ensuite lavé et séché comme dans l'exemple 13. La caractérisation de la poudre par RMN ³¹P du solide confirme le greffage (pic large centré à 26,3 ppm).

### Exemple 18

### Préparation d'une poudre d'hydroxyapatite modifiée par BDPA puis la triéthylamine

### Préparation de l'acide 12-bromododécylphosphonique (BDPA)

Le composé BDPA a été synthétisé selon le mode opératoire décrit dans l'exemple 12.

### Préparation de la poudre d'hydroxyapatite modifiée

Une solution d'acide 12-bromododécylphosphonique dans l'éthanol absolu (1 mM), est ajustée à pH = 6 par ajout d'une solution de NaOH 0,1 M. Dans un ballon de 250 ml, 200 ml de cette solution sont ajoutés à 360 mg d'hydroxyapatite (commercialisée par la société Acros, surface spécifique 67 m²/g). Le mélange est agité trois jours à température ambiante.

Après lavage à l'éthanol, cette poudre est chauffée à reflux pendant 12 heures dans l'éthanol absolu, puis elle est ensuite chauffée au reflux d'un mélange de triéthylamine (2 mL) et d'éthanol (11 mL) pendant 24 heures. Après retour à température ambiante, le solide est ensuite lavé et séché comme dans l'exemple 13. La caractérisation de la poudre par RMN ³¹P du solide confirme le greffage (pic large centré à 26,1 ppm).

### Exemple 19

### Préparation d'une poudre de carbonate de calcium modifiée par BDPA puis la triéthylamine

### Préparation de l'acide 12-bromododécylphosphonique (BDPA)

Le composé BDPA a été synthétisé selon le mode opératoire décrit dans l'exemple 12.

### Préparation de la poudre de carbonate de calcium modifiée

Une solution d'acide 12-bromododécylphosphonique dans l'éthanol absolu (1 mM) est ajustée à pH = 6 par ajout d'une solution de NaOH 0,1 M. Dans un ballon de 250 ml, 100 ml de cette solution sont ajoutés à 400 mg de CaCO₃ (surface spécifique 30 m²/g). Le mélange est agité trois jours à température ambiante.

Après lavage à l'éthanol cette poudre est chauffée à reflux pendant 12 heures dans l'éthanol absolu, puis elle est chauffée au reflux d'un mélange de triéthylamine (2 mL) et d'éthanol (11 mL) pendant 24 heures. Après retour à température ambiante, le solide est ensuite lavé et séché comme dans l'exemple 13. La caractérisation de la poudre par RMN ³¹P du solide confirme le greffage (pic large centré à 26,5 ppm).

### Exemple 20

### Tests bactériologiques

Les tests bactériologiques ont été effectués en utilisant la bactérie Pseudomona Aeruginosa.

Ces tests ont été réalisés sur les échantillons obtenus dans les exemples 1 à 4 et 6 à 12, désignés ci-après respectivement par « échantillons H, A, B, I, M, C, D, E, O, N, P », et sur 8 échantillons témoins.

Les échantillons témoins sont constitués d'une feuille de titane (échantillons F et J), d'une feuille d'acier inoxydable (échantillons G, Q), d'une feuille de titane modifiée par du MDPA (échantillon K), d'une feuille de titane modifiée par du APPE (échantillon L), d'une feuille de titane modifiée par du AEADPE (échantillon R) et d'une feuille d'acier inoxydable modifiée par du BDPA (échantillon S). Les feuilles servant de témoin modifiées par MDPA, par APPE, par AEADPE et par BDPA l'ont été dans les mêmes conditions opératoires que celles décrites respectivement dans les exemples 1, 4, 6 et 12. Toutes les feuilles servant de témoin ont été, avant d'être utilisées pour les tests, immergées dans 5 ml d'éthanol absolu.

Les échantillons (feuilles de dimensions 1 cm x 1 cm pour les échantillons A à G et feuilles de dimensions 1,8 cm x 1,8 cm pour les échantillons H à S) sont placés verticalement dans les puits d'une plaque de culture.

On introduit un milieu de culture pour bactéries (de type Muller Hinton) puis une suspension homogène de bactéries (de densité optique à 600 nm égale à 0,05).

La plaque de culture est placée pendant 72 heures dans une étuve à 37°C sous CO₂ (atmosphère humide). Un film de bactéries se forme à la surface des échantillons.

Après lavage à l'eau pour éliminer les bactéries non adhérentes qui ne forment pas le biofilm, les échantillons sont colorés au cristal violet (qui est un colorant se fixant sélectivement sur les bactéries), puis rincés abondamment à l'eau pour éliminer l'excès de cristal violet. On ajoute ensuite 1 ml de DMSO pour solubiliser le cristal violet. La mesure de la densité optique à 600 nm des solutions de colorant dans le DMSO permet de quantifier les bactéries provenant du biofilm.

Les résultats des tests bactériologiques sont regroupés dans le tableau ci-dessous.

| **Echantillon** | **Densité optique à 600 nm normalisée à 100 % pour support (Ti ou Acier) non modifié** | **Diminution par rapport au support (Ti ou Acier) non modifié** |
|---|---|---|
| A | 34,8 | -65% |
| (Ti/MDPA/ions Ag⁺) | | |
| B | 20,5 | -80% |
| (Ti/AEPA/NO) | | |
| C | 17,2 | -83% |
| (Acier/MDPA/ions Ag⁺) | | |
| D | 11,4 | -89% |
| (Acier/AEPA/NO) | | |
| E | 20,2 | -80% |
| (Acier/APPE/NO) | | |
| F | 100 | / |
| (Ti) | | |
| G | 100 | / |
| (Acier) | | |
| H | 26,8 | -73% |
| (Ti/MDPA/nanoparticules | | |
| Ag) | | |
| I | 25,4 | -75% |
| (Ti/APPE/NO) | | |
| J | 100 | / |
| (Ti) | | |
| K | 90 | -10% |
| (Ti/MDPA) | | |
| L | 101 | +1% |
| (Ti/APPE) | | |
| M | 17,3 | -83% |
| (Ti/AEADPE/NO) | | |
| N | 31,7 | -68% |
| (Acier/BMimPPE) | | |
| O | 28, 6 | -71% |
| (Acier/BMimPPA) | | |
| P | 36,5 | -64% |
| (Acier/BDPA/Et₃N) | | |
| Q | 100 | / |
| (Acier) | | |
| R | 86,4 | -14% |
| (Ti/AEADPE) | | |
| S | 96,8 | -3,2% |
| (Acier/BDPA) | | |

Pour tous les échantillons libérant des ions Ag⁺ ou du monoxyde d'azote (échantillons A, B, C, D, E, H, I, M), on observe une très nette diminution (65 à 89%) du nombre de bactéries adhérentes formant un biofilm, par rapport aux échantillons témoins (supports Ti ou acier non modifiés). De plus, pour les échantillons K, L et R, avant réaction avec les ions Ag+, les nanoparticules d'argent ou le NO, la diminution est beaucoup plus faible (<15%). Cela confirme que l'activité anti-bactérienne provient de la libération en milieu physiologique des ions Ag⁺ ou du monoxyde d'azote présents sur les substrats modifiés par le procédé selon l'invention.

Pour les échantillons présentant des fonctions ammonium quaternaires (échantillons N, O, P), la diminution du nombre de bactéries adhérentes formant un biofilm, par rapport aux échantillons témoins (supports Ti ou acier non modifiés), est comprise entre 64 et 68%. La diminution est très faible pour l'échantillon S, modifié uniquement par le BDPA. L'activité anti-bactérienne provient donc bien de la présence des fonctions ammonium quaternaires sur les substrats modifiés par le procédé selon l'invention.

## Revendications

1. Procédé de modification d'un substrat inorganique visant à lui conférer des propriétés anti-microbiennes, ledit procédé consistant à greffer en une ou plusieurs étapes sur une surface dudit substrat des groupements ayant des propriétés anti-microbiennes intrinsèques ou des groupements capables de libérer des espèces ayant des propriétés anti-microbiennes, le greffage étant effectué par l'intermédiaire d'un agent de couplage organophosphoré, **caractérisé en ce que** :
- le substrat est choisi parmi les métaux, les carbonates métalliques et les phosphates métalliques ;
- l'agent de couplage organophosphoré est choisi parmi les acides phosphoniques et les phosphonates de formule RPO(OX)₂, les bis-phosphonates de formule RR'[PO(OX)₂]₂, les acides phosphiniques et les phosphinates de formule RR'PO(OX), les monoalkyle phosphates de formule ROPO(OX)₂, et les dialkyle phosphates de formule (RO)(R'O)PO(OX), dans lesquelles :
• X représente un atome d'hydrogène ou un groupe choisi parmi les ions métalliques, les ions ammonium, les groupements alkyle ou aryle comportant de 1 à 6 atomes de carbone et les groupements trialkylsilyles comportant de 1 à 6 atomes de carbone ;
• R est un groupement organique choisi parmi :
- les groupements qui ont des propriétés anti-microbiennes intrinsèques ;
- les groupements qui sont capables de libérer des espèces ayant des propriétés anti-microbiennes choisis parmi les groupements libérant du monoxyde d'azote et les groupements libérant des acides carboxyliques ;
- les groupements R" qui, après modification, ont des propriétés anti-microbiennes intrinsèques ou sont capables de libérer des espèces ayant des propriétés anti-microbiennes, lesdits groupements R étant choisis parmi les groupements -(CH₂)ₙ-SH, -(CH₂)ₙ-CN,
- (CH₂)ₙ-NH₂, -(CH₂)ₙ-[NH-(CH₂)ₙ]ₙ-NH₂, -(CH₂)ₙ-NMe₂,
- (CH₂)ₙ-Hal, Hal représentant un atome d'halogène, n étant compris entre 1 et 20, n étant compris entre 1 et 5, n étant compris entre 1 et 10;
R' est un atome d'hydrogène, un groupement hydroxyle ou un groupement organique répondant à la définition desdits groupements R ;
- l'agent de couplage organophosphoré est lié à la surface du substrat par l'intermédiaire de liaisons M-O-P dans lesquelles M représente le métal du substrat, lesdites liaisons provenant de la condensation des groupements P-OX et/ou de la coordination des groupements phosphoryle P=O.

2. Procédé selon la revendication 1, **caractérisé en ce que** les groupements ayant des propriétés anti-microbiennes intrinsèques sont choisis parmi les groupements comportant une ou plusieurs fonctions ammonium, pyridinium, imidazolium, phosphonium, sulfate, biguanide, carbanilide, amidine, pyrimidine, hydroxyquinoléine, acide carboxylique, des peptides, des enzymes.

3. Procédé selon la revendication 2, **caractérisé en ce que** l'agent de couplage est le 3-(bromure de N-méthyl imidazolium) propylphosphonate de diéthyle (BMImPPE) ou l'acide 3-(bromure de N-méthyl imidazolium) propylphosphonique (BMImPPA).

4. Procédé selon la revendication 1, **caractérisé en ce que** les groupements capables de libérer des espèces ayant des propriétés anti-microbiennes sont des groupements libérant du monoxyde d'azote choisis parmi les groupements diazénium diolates et les groupements oxynitroxy ou des groupements libérant des acides carboxyliques choisis parmi les groupements esters ou amides.

5. Procédé selon la revendication 1, **caractérisé en ce que** les groupements R" sont modifiés par immersion dans une solution de nanoparticules d'argent ou dans une solution de AgNO₃.

6. Procédé selon la revendication 1, **caractérisé en ce que** les groupements R" sont choisis parmi les groupements -(CH₂)ₙ-NH₂ et -(CH₂)ₙ-NH-(CH₂)₂-NH₂, et **en ce que** lesdits groupements R" sont modifiés par mise sous atmosphère de monoxyde d'azote gazeux.

7. Procédé selon la revendication 1, **caractérisé en ce que** les groupements R" sont :
- des groupements -(CH₂)ₙ-NMe₂, et lesdits groupements R" sont modifiés par chauffage dans une solution de bromure d'alkyle ; ou
- des groupements -(CH₂)ₙ-Br, et lesdits groupements R" sont modifiés par chauffage dans une solution de triéthyle amine.

8. Procédé selon la revendication 1, **caractérisé en ce que** l'agent de couplage est choisi parmi l'acide 12-mercaptododécylphosphonique (MDPA), l'acide 2-aminoéthylphosphonique (AEPA), le diéthyle 3-aminopropylphosphonate (APPE), l'acide 12-bromododécylphosphonique (BDPA), le 3(N,N-diméthylamino)dodécylphosphonate de diéthyle (DMADPE), le 3-(bromure de N-méthyl imidazolium) propylphosphonate de diéthyle (BMImPPE), l'acide 3-(bromure de N-méthyl imidazolium) propylphosphonique (BMImPPA) et le 12-N-(aminoéthyl)-aminododécylphosphonate de diéthyle (AEADPE).

9. Procédé selon la revendication 1, **caractérisé en ce que** le substrat à traiter est choisi parmi un acier inoxydable, un acier galvanisé, du titane, du titane recouvert d'hydroxyapatite, un alliage à base de titane ou de chrome, de l'aluminium, du cuivre, un carbonate de calcium, de l'hydroxyapatite.

10. Procédé selon la revendication 1, **caractérisé en ce que** l'agent de couplage est greffé sur la surface dudit substrat par immersion dudit substrat dans une solution contenant ledit agent de couplage.

11. Substrat inorganique modifié obtenu par le procédé selon l'une quelconque des revendications 1 à 10, **caractérisé en ce qu'**il est choisi parmi un métal, un carbonate métallique, un phosphate métallique, et **en ce qu'**il a une surface à laquelle sont liés des groupes organophosphorés ayant au moins un substituant organique R¹, ledit substituant organique R¹ possédant des propriétés anti-microbiennes ou étant susceptible de libérer, en milieu physiologique, une espèce possédant des propriétés anti-microbiennes, **caractérisé en ce que** les groupes organophosphorés sont de type phosphonate R¹PO₁₋ₓ(OX)_{2-y}(OM)_{x+y}, bis-phosphonate R¹R²[PO₁₋ₓ(OX)_{2-y}(OM)_{x+y}]₂, phosphinate R¹R²PO₁₋ₓ(OX)_{1-z}(OM)_{x+z}, ou phosphate R¹OPO₁₋ₓ(OX)_{2-y}(OM)_{x+y} ou diester (R¹O)(R²O)PO R¹R²PO₁₋ₓ(OX)_{1-z}(OM)_{x+z}, dans lesquels :
X représente un atome d'hydrogène ou un groupe choisi parmi les ions métalliques, les ions ammonium, les groupements alkyle ou aryle comportant de 1 à 6 atomes de carbone et les groupements trialkylsilyles comportant de 1 à 6 atomes de carbone ;
R¹ est un groupement organique choisi parmi :
- les groupements qui ont des propriétés anti-microbiennes intrinsèques ;
- les groupements qui sont capables de libérer des espèces ayant des propriétés anti-microbiennes, lesdits groupements étant choisis parmi les groupements libérant des ions métalliques, les groupements libérant du monoxyde d'azote ou les groupements libérant des acides carboxyliques ;
R² est un atome d'hydrogène, un groupement hydroxyle ou un groupement organique pouvant éventuellement faire partie des groupements R¹ définis ci-dessus ;
M représente le métal du substrat ;
x vaut 0 ou 1 ;
y vaut 0, 1, ou 2 ;
Z vaut 0 ou 1,
et **en ce que** lesdits groupes organophosphorés sont liés à la surface du substrat par l'intermédiaire de liaisons M-O-P dans lesquelles M représente le métal du substrat, lesdites liaisons provenant de la condensation des groupements P-OX et/ou de la coordination des groupements phosphoryle P=O.

12. Substrat selon la revendication 11, **caractérisé en ce qu'**il porte à sa surface des groupements possédant des propriétés anti-microbiennes ou des groupements susceptibles de libérer des éléments possédant des propriétés anti-microbiennes choisis parmi les groupements libérant des ions métalliques, les groupements libérant du monoxyde d'azote ou les groupements libérant des acides carboxyliques.

13. Substrat selon la revendication 11, **caractérisé en ce que** les groupements libérant des ions métalliques sont choisis parmi les groupements portant une ou plusieurs fonctions amine, acide, thiol, cyano, ou disulfure, complexant des ions ou des particules métalliques.

14. Substrat selon la revendication 11, **caractérisé en ce que** les groupements libérant des acides carboxyliques sont des groupements esters ou amides.

15. Substrat selon la revendication 11, **caractérisé en ce que** le groupement R¹ est :
- un groupement -(CH₂)₁₂-SH ayant fixé des ions Ag⁺ ou des particules d'argent ;
- un groupement -(CH₂)₁₂-Br ayant fixé une amine tertiaire ;
- un groupement -(CH₂)₁₂-NMe₂ ayant fixé du bromure d'alkyle ;
- un groupement -(CH₂)₂-NH₂ ayant fixé du monoxyde d'azote ;
- un groupement -(CH₂)₃-NH₂ ayant fixé du monoxyde d'azote ;
- un groupement - (CH₂)₁₂-NH-(CH₂)₂-NH₂ ayant fixé du monoxyde d'azote ; ou
- un groupement -(CH₂)₃-C₃H₃N₂MeBr.

16. Substrat selon la revendication 11, **caractérisé en ce qu'**il est constitué d'une feuille de titane ou d'une feuille d'acier inoxydable, ladite feuille étant modifiée par MDPA et des ions Ag⁺ ; par AEPA et du monoxyde d'azote NO ; ou par APPE et du monoxyde d'azote NO.

17. Substrat selon la revendication 11, **caractérisé en ce qu'**il est constitué d'une feuille de titane modifiée par MDPA et des particules d'argent, ou par AEADPE et du monoxyde d'azote NO.

18. Substrat selon la revendication 11, **caractérisé en ce qu'**il est constitué par :
- une feuille de titane ou une feuille d'acier inoxydable, ou une poudre d'hydroxyapatite, ou une poudre de carbonate de calcium, ladite feuille ou ladite poudre étant modifiée par BMImPPE ;
- une feuille d'acier inoxydable, modifiée par BMImPPA ; ou
- une feuille d'acier inoxydable, une poudre d'hydroxyapatite ou une poudre de carbonate de calcium, ladite feuille ou ladite poudre étant modifiée par BDPA et de la triéthylamine.

## Patentansprüche

1. Verfahren zur Veränderung eines anorganischen Substrats, das darauf abzielt, ihm antimikrobielle Eigenschaften zu verleihen, wobei das Verfahren darin besteht, in einem oder mehreren Schritten auf die Oberfläche des Substrats Gruppen zu pfropfen, die inhärente antimikrobielle Eigenschaften haben oder Gruppen, die imstande sind, Spezies freizusetzen, die antimikrobielle Eigenschaften haben, wobei das Pfropfen mit Hilfe eines Organophosphor-Kopplungsmittels durchgeführt wird, **dadurch gekennzeichnet, dass**:
- das Substrat aus den Metallen, den Metallkarbonaten und den Metallphosphaten ausgewählt ist,
- das Organophosphor-Kopplungsmittel aus den Phosphonsäuren und den Phosphonaten der Formel RPO(OX)₂, den Bis-Phosphonaten der Formel RR'[PO(OX)₂]₂, den Phosphinsäuren und den Phosphinaten der Formel RR'PO(OX), den Monoalkylphosphaten der Formel ROPO(OX)₂ und den Dialkylphosphaten der Formel (RO)(R'O)PO(OX) ausgewählt ist, in welchen:
• X ein Wasserstoffatom oder eine Gruppe darstellt, die aus den Metallionen, den Ammoniumionen, den Alkyl- oder Aryl-Gruppen mit 1 bis 6 Kohlenstoffatomen und den Trialkylsilylgruppen mit 1 bis 6 Kohlenstoffatomen ausgewählt ist,
• R eine organische Gruppe ist, die ausgewählt ist aus:
- den Gruppen, die inhärente antimikrobielle Eigenschaften haben,
- den Gruppen, die imstande sind, Spezies freizusetzen, die antimikrobielle Eigenschaften haben, ausgewählt aus den Gruppen, die Stickstoffmonoxid freisetzen und den Gruppen, die Carboxylsäuren freisetzen,
- den Gruppen R", die nach Veränderung inhärente antimikrobielle Eigenschaften haben oder imstande sind, Spezies freizusetzen, die antimikrobielle Eigenschaften haben, wobei die Gruppen R aus den Gruppen -(CH₂)ₙ-SH, -(CH₂)ₙ-CN, -(CH₂)ₙ-NH₂, -(CH₂)ₙ-[NH-(CH₂)ₙ]ₙ-NH₂, -(CH₂)ₙ-NMe₂, -(CH₂)ₙ-Hal ausgewählt sind, wobei Hal ein Halogenatom darstellt, wobei n zwischen 1 und 20 inklusive ist, wobei n zwischen 1 und 5 inklusive ist, wobei n zwischen 1 und 10 inklusive ist,
• R' ein Wasserstoffatom, eine Hydroxylgruppe oder eine organische Gruppe ist, die der Definition der Gruppen R entspricht,
- dass das Organophosphor-Kopplungsmittel an der Oberfläche des Substrats mit Hilfe von M-O-P-Bindungen gekoppelt ist, in welchen M das Metall des Substrats darstellt, wobei die Bindungen aus der Kondensation der Gruppen P-OX und/oder der Koordination der Phosphorylgruppen P=O resultieren.

2. Verfahren nach Anspruch 1, **dadurch gekennzeichnet, dass** die Gruppen, die inhärente antimikrobielle Eigenschaften haben, aus den Gruppen, die eine oder mehrere Ammonium-, Pyridinium-, Imidazolium-, Phosphonium-, Sulfat-, Biguanid-, Carbanilid-, Amidin-, Pyrimidin-, Hydroxyquinolein-, Carboxylsäurefunktionen haben, den Peptiden, den Enzymen ausgewählt sind.

3. Verfahren nach Anspruch 2, **dadurch gekennzeichnet, dass** das Kopplungsmittel das 3-(N-methylimidazoliumbromid)-diethylpropylphosphonat (BMImPPE) oder die 3-(N-methylimidazoliumbromid)-propylphosphonsäure (BMImPPA) ist.

4. Verfahren nach Anspruch 1, **dadurch gekennzeichnet, dass** die Gruppen, die imstande sind, Spezies freizusetzen, die antimikrobielle Eigenschaften haben, Gruppen sind, die Stickstoffmonoxid freisetzen, ausgewählt aus den Diazeniumdiolatgruppen und den Oxynitroxygruppen, oder Gruppen, die Carboxylsäuren freisetzen, ausgewählt aus den Ester- oder Amidgruppen.

5. Verfahren nach Anspruch 1, **dadurch gekennzeichnet, dass** die Gruppen R" durch Eintauchen in eine Silbernanopartikellösung oder in eine AgNO₃-Lösung modifiziert werden.

6. Verfahren nach Anspruch 1, **dadurch gekennzeichnet, dass** die Gruppen R" aus den Gruppen -(CH₂)ₙ-NH₂ und -(CH₂)ₙ-NH-(CH₂)₂-NH₂ ausgewählt sind und dass die Gruppen R" durch Versetzen in eine gasförmige Stickstoffmonoxidatmosphäre modifiziert werden.

7. Verfahren nach Anspruch 1, **dadurch gekennzeichnet, dass** die Gruppen R" sind:
- Gruppen -(CH₂)ₙ-NMe₂, und die Gruppen R" durch Erhitzen in einer Alkylbromidlösung modifiziert werden, oder
- Gruppen Gruppen -(CH₂)ₙ-Br, und die Gruppen R" durch Erhitzen in einer Triethylaminlösung modifiziert werden.

8. Verfahren nach Anspruch 1, **dadurch gekennzeichnet, dass** das Kopplungsmittel aus der 12-Mercaptododecylphosphonsäure (MDPA), der 2-Aminoethylphosphonsäure (AEPA), dem Diethyl-3-aminopropylphosphonat (APPE), der 12-Bromdodecylphosphonsäure (BDPA), dem 3-(N,N-dimethylamin)-diethyldodecylphosphonat (DMADPE), dem 3-(N-methylimidazoliumbromid)-diethylpropylphosphonat (BMImPPE), der 3-(N-methylimidazoliumbromid)-propylphosphonsäure (BMImPPA) und dem 12-N-(aminoethyl)-diethylaminododecylphosphonat (AEADPE) ausgewählt ist.

9. Verfahren nach Anspruch 1, **dadurch gekennzeichnet, dass** das zu bearbeitende Substrat aus einem rostfreien Stahl, einem galvanisierten Stahl, Titan, mit Hydroxyapatit überzogenem Titan, einer Legierung auf der Basis von Titan oder Chrom, Aluminium, Kupfer, einem Calciumcarbonat, Hydroxyapatit ausgewählt ist.

10. Verfahren nach Anspruch 1, **dadurch gekennzeichnet, dass** das Kopplungsmittel auf die Oberfläche des Substrats durch Eintauchen des Substrats in eine Lösung gepfropft wird, die das Kopplungsmittel enthält.

11. Modifiziertes anorganisches Substrat, erhalten durch das Verfahren nach einem der Ansprüche 1 bis 10, **dadurch gekennzeichnet, dass** es aus einem Metall, einem Metallcarbonat, einem Metallphosphat ausgewählt ist, und dass es eine Oberfläche hat, an die Organophosphorgruppen gebunden sind, die mindestens einen organischen Substituenten R¹ haben, wobei der organische Substituent R¹ antimikrobielle Eigenschaften besitzt oder imstande ist, in physiologischem Medium eine Spezies freizusetzen, die antimikrobielle Eigenschaften besitzt, **dadurch gekennzeichnet, dass** die Organophosphorgruppen von Typ Phosphonat R¹PO₁₋ₓ(OX)_{2-y}(OM)_{x+y}, Bis-phosphonat R¹R²[PO₁₋ₓ(OX)_{2-y}(OM)_{x+y}]₂, Phosphinat R¹R²PO₁₋ₓ(OX)_{l-z}(OM)_{x+z} Phosphate R¹OPO₁₋ₓ(OX)_{2-y}(OM)_{x+y} oder Diester (R¹O)(R²O)POR¹R²PO₁₋ₓ(OX)_{1-z}(OM)_{x+z} sind, in welchen:
X ein Wasserstoffatom oder eine Gruppe darstellt, die aus den Metallionen, den Ammoniumionen, den Alkyl- oder Aryl-Gruppen mit 1 bis 6 Kohlenstoffatomen und den Trialkylsilylgruppen mit 1 bis 6 Kohlenstoffatomen ausgewählt ist,
R eine organische Gruppe ist, die ausgewählt ist aus:
- den Gruppen, die inhärente antimikrobielle Eigenschaften haben,
- den Gruppen, die imstande sind, Spezies freizusetzen, die antimikrobielle Eigenschaften haben, wobei die Gruppen aus den Gruppen ausgewählt sind, die Metallionen freisetzen, den Gruppen, die Stickstoffmonoxid freisetzen oder den Gruppen, die Carboxylsäuren freisetzen,
R² ein Wasserstoffatom, eine Hydroxylgruppe oder eine organische Gruppe ist, die eventuell Teil der hier oben definierten Gruppen R' ist,
M das Metall des Substrats darstellt,
x 0 oder 1 entspricht,
y 0, 1 oder 2 entspricht,
Z 0 oder 1 entspricht,
und dass die Organophosphor-Gruppen an der Oberfläche des Substrats mit Hilfe von M-O-P-Bindungen gekoppelt sind, in welchen M das Metall des Substrats darstellt, wobei die Bindungen aus der Kondensation der Gruppen P-OX und/oder der Koordination der Phosphorylgruppen P=O resultieren.

12. Substrat nach Anspruch 11, **dadurch gekennzeichnet, dass** es auf seiner Oberfläche Gruppen trägt, die antimikrobielle Eigenschaften besitzen, oder Gruppen, die imstande sind, Elemente freizusetzen, die antimikrobielle Eigenschaften besitzen, ausgewählt aus den Gruppen, die Metallionen freisetzen, Gruppen, die Stickstoffmonoxid freisetzen oder Gruppen, die Carboxylsäuren freisetzen.

13. Substrat nach Anspruch 11, **dadurch gekennzeichnet, dass** die Gruppen, die Metallionen freisetzen, aus den Gruppen ausgewählt sind, die eine oder mehrere Amin-, Säure-, Thiol-, Cyano- oder Disulfidfunktionen tragen, die Ionen oder Metallpartikel komplexieren.

14. Substrat nach Anspruch 11, **dadurch gekennzeichnet, dass** die Gruppen, die Carboxylsäuren freisetzen, Ester- oder Amidgruppen sind.

15. Substrat nach Anspruch 11, **dadurch gekennzeichnet, dass** die Gruppe R¹ ist:
- eine Gruppe -(CH₂)₁₂-SH, die Ag⁺-Ionen oder Silberpartikel fixiert hat,
- eine Gruppe -(CH₂)₁₂-Br, die ein tertiäres Amin fixiert hat,
- eine Gruppe -(CH₂)₁₂-NMe₂, die Alkylbromid fixiert hat,
- eine Gruppe -(CH₂)₂-NH₂, die Stickstoffmonoxid fixiert hat,
- eine Gruppe -(CH₂)₃-NH₂, die Stickstoffmonoxid fixiert hat,
- eine Gruppe -(CH₂)₁₂-NH-(CH₂)₂-NH₂, die Stickstoffmonoxid fixiert hat, oder
- eine Gruppe -(CH₂)₃-C₃H₃N₂MeBr.

16. Substrat nach Anspruch 11, **dadurch gekennzeichnet, dass** es von einem Titanbogen oder einem Bogen aus rostfreiem Stahl gebildet ist, wobei der Bogen von MDPA und Ag⁺-Ionen, von AEPA und Stickstoffmonoxid NO oder von APPE und Stickstoffmonoxid NO modifiziert ist.

17. Substrat nach Anspruch 11, **dadurch gekennzeichnet, dass** es von einem Titanbogen gebildet ist, der von MDPA und Silberpartikeln oder von AEADPE und Stickstoffmonoxid NO modifiziert ist.

18. Substrat nach Anspruch 11, **dadurch gekennzeichnet, dass** es gebildet ist von:
- einem Titanbogen oder einem Bogen aus rostfreiem Stahl oder einem Hydroxyapaptitpulver oder einem Calciumcarbonatpulver, wobei der Bogen oder das Pulver von BMImPPE modifiziert sind,
- einem Bogen aus rostfreiem Stahl, der von BMImPPA modifiziert ist, oder
- einen Bogen aus rostfreiem Stahl, einem Hydroxyapaptitpulver oder einem Calciumcarbonatpulver, wobei der Bogen oder das Pulver von BDPA und Triethylamin modifiziert ist.

## Claims

1. A method to modify an inorganic substrate intended to impart antimicrobial properties thereto whereby, in one or more steps, groups having intrinsic antimicrobial properties or groups capable of releasing species having antimicrobial properties are grafted onto a surface of said substrate , grafting being conducted via an organophosphorus coupling agent, **characterized in that**:
- the substrate is selected from among metals, metal carbonates and metal phosphates;
- the organophosphorus coupling agent is selected from among phosphonic acids and phosphonates of formula RPO(OX)₂, bis-phosphonates of formula RR'[PO(OX)₂]₂, phosphinic acids and phosphinates of formula RR'PO(OX), monoalkyl phosphates of formula ROPO(OX)₂ and dialkyl phosphates of formula (RO)(R'O)PO(OX), wherein:
• X is a hydrogen atom or a group selected from among metal ions, ammonium ions, alkyl or aryl groups having 1 to 6 carbon atoms and trialkylsilyl groups having 1 to 6 carbon atoms;
• R is an organic group selected from among:
- groups having intrinsic antimicrobial properties;
- groups capable of releasing species having antimicrobial properties selected from among groups releasing nitrogen monoxide and groups releasing carboxylic acids;
- R" groups which, after modification, have intrinsic antimicrobial properties or are capable of releasing species having antimicrobial properties, said R groups being selected from among -(CH₂)ₙ-SH, -(CH₂)ₙ-CN, -(CH₂)ₙ-NH₂, -(CH₂)ₙ-[NH-(CH₂)ₙ]ₙ-NH₂, -(CH₂)ₙ-NMe₂, -(CH₂)ₙ-Hal groups, Hal being a halogen atom, n is between 1 and 20, n is between 1 and 5, n is between 1 and 10;
R' is a hydrogen atom, a hydroxyl group or an organic group meeting the definition of said R groups;
- the organophosphorus coupling agent is bonded to the surface of the substrate via M-O-P bonds wherein M is the metal of the substrate, said bonds being derived from condensation of the P-OX groups and/or from coordination of the phosphoryl groups P=O.

2. The method according to claim 1, **characterized in that** the groups having intrinsic antimicrobial properties are selected from among groups having one or more of the following functions: ammonium, pyridinium, imidazolium, phosphonium, sulfate, biguanide, carbanilide, amidine, pyrimidine, hydroxyquinoline, carboxylic acid, peptides, enzymes.

3. The method according to claim 2, **characterized in that** the coupling agent is diethyl 3-(N-methylimidazolium bromide) propylphosphonate (DMImBPP) or 3-(N-methylimidazolium bromide) propylphosphonic acid (MImBPPA).

4. The method according to claim 1, **characterized in that** the groups capable of releasing species having antimicrobial properties are groups releasing nitrogen monoxide selected from among diazenium diolate groups and oxynitroxy groups or groups releasing carboxylic acids selected from among ester or amide groups.

5. The method according to claim 1, **characterized in that** the R" groups are modified by immersion in a solution of silver nanoparticles or in an AgNO₃ solution.

6. The method according to claim 1, **characterized in that** the R" groups are selected from among -(CH₂)ₙ-NH₂ and -(CH₂)ₙ-NH-(CH₂)₂-NH₂ groups, and **in that** said R" groups are modified by placing in a gaseous nitrogen monoxide atmosphere.

7. The method according to claim 1, **characterized in that** the R" groups are:
- -(CH₂)ₙ-NMe₂ groups, and said R" groups are modified by heating in an alkyl bromide solution; or
- -(CH₂)ₙ-Br groups, and said R" groups are modified by heating in a triethyl amine solution.

8. The method according to claim 1, **characterized in that** the coupling agent is selected from among 12-mercaptododecylphosphonic acid (MDPA), 2-aminoethyl-phosphonic acid (AEPA), diethyl (3-aminopropyl)phosphonate (3-APP), 12-bromododecylphosphonic acid (BDPA), diethyl 3(N,N-dimethylamino)dodecylphosphonate (DDMADP), diethyl 3-(N-methylimidazolium bromide) propylphosphonate (DMImBPP), 3-(N-methylimidazolium bromide) propylphosphonic acid (MImBPPA) and diethyl 12-N-(aminoethyl)-aminododecylphosphonate (DAEADP).

9. The method according to claim 1, **characterized in that** the substrate to be treated is selected from among stainless steel, galvanized steel, titanium, hydroxyapatite-coated titanium, titanium- or chromium-based alloy, aluminium, copper, calcium carbonate, hydroxyapatite.

10. The method according to claim 1, **characterized in that** the coupling agent is grafted onto the surface of said substrate via immersion of said substrate in a solution containing said coupling agent.

11. A modified inorganic substrate obtained using the method according to any of claims 1 to 10, **characterized in that** it is selected from among a metal, a metal carbonate, a metal phosphate, and **in that** organophosphorus groups are bonded onto the surface thereof having at least one organic substituent R¹, said organic substituent R¹ having antimicrobial properties or being capable, in a physiological medium, of releasing a species having antimicrobial properties, **characterized in that** the organophosphorus groups are of the type ^{R1}PO₁₋ₓ(OX)_{2-y}(OM)_{x+y} phosphonate, R¹R²[PO₁₋ₓ(OX)_{2-y}(OM)_{x+y}]₂ bis-phosphonate, R¹R²PO₁₋ₓ(OX)_{1-z}(OM)_{x+z} phosphinate or R¹OPO₁₋ₓ(OX)_{2-y}(OM)_{x+y} phosphate or (R¹O)(R²O)POR¹R²PO₁₋ₓ (OX)_{1-z}(OM)x_{+z} diester, wherein:
X is a hydrogen atom or group selected from among metal ions, ammonium ions, alkyl or aryl groups having 1 to 6 carbon atoms and trialkylsilyl groups having 1 to 6 carbon atoms;
R¹ is an organic group selected from among:
- groups having intrinsic antimicrobial properties;
- groups capable of releasing species having antimicrobial properties, said groups being selected from among groups releasing metal ions, groups releasing nitrogen monoxide or groups releasing carboxylic acids;
R² is a hydrogen atom, hydroxyl group or organic group optionally belonging to the R¹ groups defined above;
M is the metal of the substrate;
x equals 0 or 1;
y equals 0, 1 or 2;
z equals 0 or 1,
and **in that** said organophosphorus groups are bonded to the surface of the substrate via M-O-P bonds wherein M is the metal of the substrate, said bonds being derived from condensation of the P-OX groups and/or from coordination of the phosphoryl groups P=O.

12. The substrate according to claim 11, **characterized in that**, on its surface, it carries groups having antimicrobial properties or groups able to release elements having antimicrobial properties selected from among groups releasing metal ions, groups releasing nitrogen monoxide or groups releasing carboxylic acids.

13. The substrate according to claim 11, **characterized in that** the groups releasing metal ions are selected from among groups carrying one or more amine, acid, thiol, cyano or disulfide functions, complexing metal ions or particles.

14. The substrate according to claim 11, **characterized in that** the groups releasing carboxylic acids are ester or amide groups.

15. The substrate according to claim 11, **characterized in that** the R¹ group is either a:
• -(CH₂)₁₂-SH group that has fixed Ag⁺ ions or silver particles;
• -(CH₂)₁₂-Br group that has fixed a tertiary amine;
• - (CH₂)₁₂-NMe₂ group that has fixed alkyl bromide;
• -(CH₂)₂-NH₂ group that has fixed nitrogen monoxide;
• -(CH₂)₃-NH₂ group that has fixed nitrogen monoxide;
• - (CH₂) ₁₂-NH- (CH₂) ₂-NH₂ group that has fixed nitrogen monoxide; or
• - (CH₂)₃-C₃H₃N₂MeBr group.

16. The substrate according to claim 11, **characterized in that** it is composed of titanium foil or stainless steel foil, said foil being modified by MDPA and Ag⁺ ions; by AEPA and nitrogen monoxide NO; or by 3-APP and nitrogen monoxide NO.

17. The substrate according to claim 11, **characterized in that** it is composed of titanium foil modified by MDPA and silver particles, or by DAEADP and nitrogen monoxide NO.

18. The substrate according to claim 11, **characterized in that** it is composed of:
- titanium foil or stainless steel foil, or hydroxyapatite powder or calcium carbonate powder, said foil or said powder being modified by DMImBPP;
- stainless steel foil modified by MimBPPA; or
- stainless steel foil, hydroxyapatite powder or calcium carbonate powder, said foil or said powder being modified by BDPA and trimethylamine.
